# EUROPÄISCHE PATENTSCHRIFT

(11) **EP 0 910 313 B1**
(45) Veröffentlichungstag und Bekanntmachung des Hinweises auf die Patenterteilung: **05.11.2003**
(21) Anmeldenummer: 97931640.3
(22) Anmeldetag: 18.06.1997
(51) Int. Cl.: A61F 2/24

(54) **PROTHETISCHE MITRAL-HERZKLAPPE**
MITRAL VALVE PROSTHESIS
VALVULE MITRALE PROTHETIQUE

(30) Priorität: 24.06.1996 DE 19625202
(43) Veröffentlichungstag der Anmeldung: 28.04.1999
(73) Patentinhaber: ADIAM life science AG, 41812 Erkelenz (DE)
(72) Erfinder: JANSEN, Josef, D-50937 Köln (DE)
(74) Vertreter: Vomberg, Friedhelm, Dipl.-Phys.
(86) Internationale Anmeldenummer: DE9701297
(87) Internationale Veröffentlichungsnummer: WO97049355

(56) Entgegenhaltungen:
- EP-A- 0 183 904
- WO-A-91/19465
- FR-A- 2 548 888
- US-A- 4 218 783
- US-A- 5 156 621

## Beschreibung

Die Erfindung betrifft eine prothetische Mitral-Herzklappe, bestehend aus einem Stützgehäuse (Stent) mit einem Basisring, der zwei im wesentlichen in Ringachsrichtung weisende, über bogenförmige, der Befestigung zweier flexibler Segel dienender Wandungen verbundene Pfosten trägt, deren freie Enden eine Innenauflage für das Segel bilden.

Solche Mitral-Herzklappen werden mittels eines an einem Basisring befestigten Nahtringes im Körpergewebe eingenäht.

Die ersten nach dem Stand der Technik bekannten Mitral-Herzklappen besaßen ein kreisrohrförmiges Klappengehäuse, in dem zwei Segel angeordnet waren, die eine aus einer Zylinderoberfläche herausgeschnittene Form aufwiesen und die sich im geschlossenen Zustand gegeneinander abstützten und im geöffneten Zustand gegen die Zylinderwandung des Klappengehäuses legten. Wie bereits in der DE 27 42 681 B2 erwähnt wird, ist das Schließvermögen solcher Herzklappen nicht optimal. Darüber hinaus wurde ein relativ langes Klappengehäuse benötigt. Um hier Abhilfe zu schaffen, wird in der vorgenannten Druckschrift vorgeschlagen, statt zweier Segel nur eine einzige Membran zu verwenden, die demjenigen Teil der Oberfläche eines elliptischen Zylinders entspricht, der von einem Kreiszylinder herausgeschnitten wird. Das Klappengehäuse soll als von dem elliptischen Zylinder und einem Winkel von 90° geschnittenes Kreisrohr ausgebildet sein, wobei die Membran entlang des halben Umfangs dieser Schnittkante zwischen deren beiden Extrempunkten, die den eingangs genannten Pfosten entsprechen, befestigt ist. Bei dieser Ausführungsform wird zwar wegen des Verzichts auf zwei Segel eine Faltung der aneinanderliegenden Segel im Schließzustand verhindert, jedoch kann ein unzureichender Klappen-Verschluß damit nicht verhindert werden.

Bei prothetischen Herzklappen stellen die variierenden physiologischen Belastungszustände in Form von unterschiedlichen Schließdruckdifferenzen, denen sich die Herzklappe anpassen muß, ein weiteres Problem dar. Durch solche Schließdruckdifferenzen werden radiale Kraftkomponenten über die Segel auf die eingangs genannten Pfosten ausgeübt, die dadurch radial zur Klappenmitte hin verformt werden. Bei steigender Schließdruckdifferenz können sich die Segel stromauf senken, ineinander einbauchen und sich entlang ihres freien Randes aneinander anlegen, was zur vollständigen Segelüberlappung und damit zur Abdichtung der Klappe erwünschtermaßen führt, jedoch bei hohen Druckdifferenzen auch zu überflüssig großen Überlappungen der freien Segelränder führen kann, wodurch unerwünschte Segel-Faltungen entstehen. Zur Verringerung von hohen Spannungen in den oberen Segelbereichen, die an die Stentspitzen angrenzen und den natürlichen Kommissuren entsprechen, ist bereits vorgeschlagen worden, die Pfosten im oberen Teil flexibel zu konstruieren. Dies kann jedoch zu unerwünschten Kriech-Erscheinungen und damit zum frühzeitigen Materialermüden führen. Um die radial nach innen zur Klappenachse gerichtete Verformung der Pfosten zu begrenzen, wird daher in der DE 42 22 610 A1 vorgeschlagen, die freien Pfostenenden starr auszubilden, insbesondere durch eine Materialanhäufung in Form einer prismatischen Innenauflage in den freien Pfostenenden, die im Querschnitt dreieckig ist. Die prismatische Innenauflage soll sich zur Stent-Basis, d.h. zum Einlaufbereich der Herzklappe hin, konkav verjüngen.

Es ist Aufgabe der vorliegenden Erfindung, die eingangs genannte Mitral-Herzklappe durch eine neue Formgebung und einen neuen konstruktiven Aufbau dahingehend zu verbessern, daß eine potentielle gegenseitige Funktionsbeeinträchtigung des Herzens und der Klappe vermieden wird.

Diese Aufgabe wird durch die prothetische Mitral-Herzklappe nach Anspruch 1 gelöst. Die beiden Halbformen bilden somit einen Stent-Körper, der der natürlichen Mitral-Klappe eines Herzens, welches eine D- oder Nieren-Form aufweist, weitgehend angenähert ist. Soweit wie z.B. in der US-A-54 15 667 sogenannte biologische Mitralklappe ohne Stent beschrieben werden, besitzen sie gegenüber der erfindungsgemäßen Mitral-Herzklappe den Unterschied, daß das aortale Segel der Seite mit geringerer Krümmung zugeordnet ist, während das murale Segel in dem Bereich liegt, der eine größere Krümmung aufweist. Die Halbformen können Halbellipsen, Hyperbeln oder andere Formen sein, bei denen als Randbedingung vorzugsweise gewährleistet sein sollte, daß die Übergangspunkte beider Hälften stetig differenzierbar sind.

Vorzugsweise liegt die Segelneigung, die durch die Lage der Verbindungslinie des Segels mit der oberen Innenkante der Wandung bestimmt ist, zwischen 25° und 45° für das weniger geneigte (aortale) Segel und zwischen 40° und 65° für das stärker geneigte (murale) Segel, jeweils relativ zur Basisgrundfläche. Das stärker geneigte Segel besitzt eine mindestens 5° größere Winkelanstellung als das weniger geneigte Segel.

Nach einer weiteren Ausgestaltung der Erfindung ist die Hauptströmungsrichtung um 10° bis 25°, vorzugsweise um 15° von der Normalen zum muralen Segel geneigt. Durch diese Maßnahme wird das Risiko der Interferenz und eine mögliche Beeinträchtigung des Stützgehäuses und der kontrahierenden Herzkammerinnenwand verringert. Die Segel bilden einen ausgeprägten trichterförmigen Öffnungskanal mit einem im Vergleich zu einer Aorten-Klappe geringeren Öffnungsquerschnitt. Die beschriebene Anordnung und Gestalt gewährleistet eine günstige physiologische Strömungsführung vom Vorhof in den Ventrikel. Die dargestellte erfindungsgemäße Herzklappe kann zudem in geringerer Bauhöhe gefertigt werden als die nach dem Stand der Technik bekannten Ausführungen. Dies gilt insbesondere im Hinblick auf im Querschnitt kreisförmige oder symmetrisch-ellipsenförmige Stützgehäuse.

In einer bevorzugten Ausführungsform stehen die Längen der halben Querachsen der Halbellipsen des Stützgehäuses in einem Verhältnis von 1,5 bis 2,5 : 1. Insbesondere bei einem Halb-Achsenverhältnis von 2 : 1 läßt sich eine weitgehend der natürlichen Mitralklappe angeordnete Form erzielen.

Die gemeinsame Längsachse der beiden unterschiedlichen Halbellipsen des Stützgehäuses besitzen eine Länge zwischen 10 mm und 45 mm.

Vorzugsweise sind die Pfosten dickengleich in die Wandungen integriert, d.h., die beschriebenen Pfosten treten gegenüber dem Wandungsbereich nicht mehr hervor, vielmehr läuft die Wandung im Bereich der vorerwähnten Pfosten nach oben hin aus, vorzugsweise zu einem spitzen oder abgeflachten Pfostenende.

Wie prinzipiell bereits in der DE 42 22 610 A1 beschrieben, können die Pfosten alternativ zu der vorbeschriebenen Ausführung prismatisch ausgebildet sein. Die Pfosten verdicken sich zu ihrem freien Ende zum genannten Stirnflächenmaß, vorzugsweise kontinuierlich. Umgekehrt verjüngen sich die Pfosten zur Basisgrundfläche hin keilförmig, wobei sie im Einlaufbereich, d.h. vor der unteren Kante des Basisringes durch Übergang in die dortige Basisring-Wanddicke, enden.

Um zu verhindern, daß sich das Stützgehäuse beim Öffnen und Schließen der Segel entsprechend den einstellenden Druckdifferenzen verformt, ist die Dicke bzw. Wandstärke der Wandung des Basisrings im Bereich zwischen den Pfosten, d.h. an der Segelbasis größer als im pfostennahen Bereich, vorzugsweise ist die Dicke um einen Faktor 1,4 bis 2,3 größer gewählt.

Um zu verhindern, daß die Klappensegel im Kommissurenbereich sehr stark beansprucht werden, ist nach einer weiteren Ausgestaltung der Erfindung die Verbindungslinie der Segel mit der oberen Innenkante der Wandung auf jeder Seite derart gelegt, daß sie in einer Ebene liegt. Durch diese Ausgestaltung der Wandungs-Stirnfläche, die der Segelbefestigung dient, werden hohe Spannungen vermieden.

Werden die Pfosten des Stützkörpers so angeordnet, daß deren Längsachse etwa in Richtung der Hauptströmungsrichtung, d.h. um 0° bis 20° gegen die Basisgrundfläche, geneigt ist, läßt sich die Mitral-Herzklappe hinsichtlich des Strömungsquerschnitts, der Bauhöhe und ihrer Stabilität weiter verbessern.

Mit der beschriebenen Mitral-Herzklappe können gegenüber den aus dem Stand der Technik bekannten Ausführungen vielfältige konstruktions- und materialbedingte Risiken vermieden werden. Durch den erfindungsgemäßen Aufbau der Mitral-Herklappe wird eine weitere Annäherung der Gestaltung an die natürliche Mitralklappe erreicht. Gegenüber den Bioprothesen als Mitralklappenersatz, der erfahrungsgemäß in 50% der Fälle eine gerinnungshemmende Medikamentation der Patienten erforderlich macht, kann die erfindungsgemäße Mitralklappenprothese medikamentenfrei funktionieren, da die geschaffene Strömungsführung durch die Kombination der Segelanstellung, des Öffnungsquerschnittes sowie der Strömungsrichtung die mechanische Blutschädigung weitgehend miminiert.

Ein Ausführungsbeispiel der Erfindung ist in den Zeichnungen dargestellt. Es zeigen:

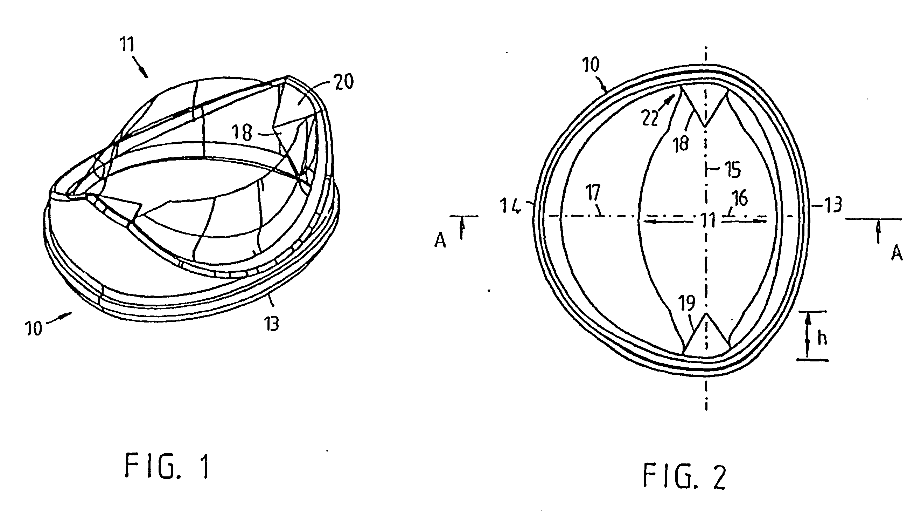

- Fig. 1: eine perspektivische Ansicht einer prothetischen Mitral-Herzklappe,
- Fig. 2: eine Draufsicht auf die Herzklappe nach Fig. 1,

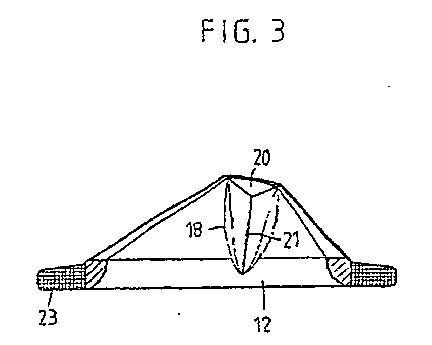

- Fig. 3: einen Schnitt entlang der Linie A-A,
- Fig. 4: eine perspektivische Ansicht einer weiteren prothetischen Mitral-Herzklappe und
- Fig. 5: eine Draufsicht auf die Herzklappe nach Fig. 4.

Die prothetische Mitral-Herzklappe besteht aus einem Stützgehäuse 10 mit zwei Segeln 11. Das Stützgehäuse 10 wird mittels eines Nahtrings 23 im Klappenannulus im Patientengewebe angenäht. Das Stützgehäuse 10 besteht aus einem Thermoplast wie Polyamid, das zu einem gering biegeelastischen Köper, beispielsweise durch Spritzgießen, hergestellt und anschließend mit einer Außenbeschichtung aus Polyurethan versehen worden ist. Das einstückige Stützgehäuse 10 weist einen Basisring 12 auf, dessen Innenkanten nach außen hin in nach dem Stand der Technik bekannter Weise abgerundet sind. Zur besseren Befestigung des Nahtringes 23 kann der Basisring am Außenmantel eine Wulst aufweisen. Die im wesentlichen zur Basisring-Grundfläche senkrechte Wandung teilt sich in eine erste Wandung 13 mit geringerer Krümmung und eine zweite Wandung 14 mit größerer Krümmung auf, die zusammen in Draufsicht auf die Basisgrundfläche betrachtet zwei Halbformen mit einer gemeinsamen Längsachse 15 bilden. Demgemäß sind auch die halben Querachsen 16 und 17 unterschiedlich lang, vorzugsweise beträgt ihr Längenverhältnis 1 : 2. Bis auf einen etwaigen Wulst im Basisring-Bereich ist der Außenmantel der Wandungen 13 und 14 gekrümmt, aber glattflächig. Entsprechendes gilt mit Ausnahme der nachfolgend noch beschriebenen Pfosten 18,19 für den Innenmantel der Wandungen 13 und 14.

Die Wanddicke der Wandungen 13 und 14 ist unterschiedlich und minimiert sich zu pfostennahen Bereichen bzw. ist im mittleren Bereich am größten, vorzugsweise ist die Wandungsdicke im mittleren Bereich zwischen den Pfosten doppelt so groß wie in dem pfostennahen Bereich. Die obere Stirnfläche der Wandungen 13 und 14, an der das Segel befestigt wird, ist nach außen abgeschrägt und verläuft im wesentlichen bis zum Bereich der Pfosten in der Form einer Schnittlinie, die sich aus einem Schnitt der jeweiligen Halbform mit einer hierzu schrägwinklig gestellten Ebene ergibt. Zum Ankleben vorgefertigter Segel liegt die Verbindungslinie der Segel mit der oberen Innenkante der Wandungen 13 und 14 in einer Ebene, die bezogen auf die Basisring-Grundfläche um einen Winkel von ca. 56° für die obere Kante der Wandung 13 bzw. einem Winkel von 41,5° für die Oberkante der Wandung 14 verläuft. Die Stirnfläche kann jedoch auch tangential zu der Ebene verlaufen, die das betreffende Segel in geschlossenem Zustand einnimmt. Die Anordnung der oberen Innenkante der Wandungen 13 und 14 in einer jeweils unter einem anderen Winkel angestellten Ebene schafft den Vorteil, daß beide Segel aus einer ebenflächigen Kunststoffolie ausgeschnitten werden können und ohne Zugbeanspruchung bzw. ohne Gefahr von Faltenbildungen an den oberen Kanten der Wandungen bis in die pfostennahen Bereiche geklebt werden können.

Als Material für die Segel kann auf die nach dem Stand der Technik bekannten Kunststoffolien, vorzugsweise aus thermoplastischen Elastomeren oder aus Kunststoff mit elastomeren Eigenschaften zurückgegriffen werden, vorzugsweise bestehen die Segel aus flexiblen Polyurethan-Folien.

Die Pfosten 18 und 19 verbreitern sich zu ihren oberen Stirnflächen 20 gleichmäßig. In einer Draufsicht auf die Innenwandung des Stützgehäuses 10 betrachtet sind die Pfosten V-förmig ausgeführt und enden keilförmig oberhalb der Basisring-Grundfläche im Einlaufbereich des Stützgehäuses 10. Die Pfostenlängsachse 21 verläuft bezogen auf die Basisring-Grundfläche nicht vertikal, sondern gegenüber einer Flächennormalen leicht geneigt, beispielsweise unter einem Winkel von 65°. Eine dementsprechende Neigung von vorzugsweise 15° besitzt auch die Stirnfläche 20 der Pfosten gegenüber der Basisring-Grundfläche. Die Pfosten 18, 19 bzw. deren Stirnflächen 20 ersetzen die bei natürlichen Klappen gegebenen Kommissurensegel und dienen mit ihren etwa gleichlangen Dreiecksschenkeln als Innenauflage für die Segel 11. In den Übergangsbereichen 22 der gleichlangen Dreiecksschenkel zu den Wandungen 13 und 14 ist das Stützgehäuse abgerundet ausgeführt. Die Segel 11 sind an der Oberkante der Wandungen 13 und 14 mit dem Stützgehäuse verklebt und sind so geschnitten, daß sie im geschlossenen Zustand an den seitlichen gleichschenkligen Pfostenrändern und in den zwischen den Pfosten 18 und 19 liegenden Bereichen etwa linienförmig an dem gegenüberliegenden Segel anliegen. Die durch die Pfosten 18 und 19 geschaffenen Kommissurenbereiche verhindern ein Durchschlagen der Segel und dienen zusammen mit den entlang der Längsachse 15 verlaufenden Überlappungsbereichen der Segel 11 zu einer Segelabstützung. Durch die in einer Ebene liegende Verbindungslinie der Segel 11 an den Wandungen 13 und 14 bis hin zu den Kommissurenbereichen wird eine gleichmäßige Kraftverteilung zwischen den Segeln 11 und dem Stützgehäuse 10 geschaffen, insbesondere werden hohe radiale Zugbeanspruchungen an den Pfostenenden bzw. in den pfostennahen Bereichen wirksam vermieden, die bei nach dem Stand der Technik bekannten Konstruktionen zu Materialermüdungen des Stützgehäuses, dem sogenannten Kriechen führten.

Der Aufbau des Stützgehäuses 10 ist der natürlichen D- oder Nierenform weitgehend angepaßt, wobei das murale Segel an der Oberkante der Wandung 13 unter einem steileren Anstellwinkel und das aortale Segel an der Wandung 14 unter einer flacheren Anstellung angeordnet sind. Hierdurch ergibt sich eine geringere Bauhöhe der Mitral-Herzklappe, deren Hauptströmungsrichtung nicht koaxial, sondern um etwa 15° hierzu geneigt verläuft.

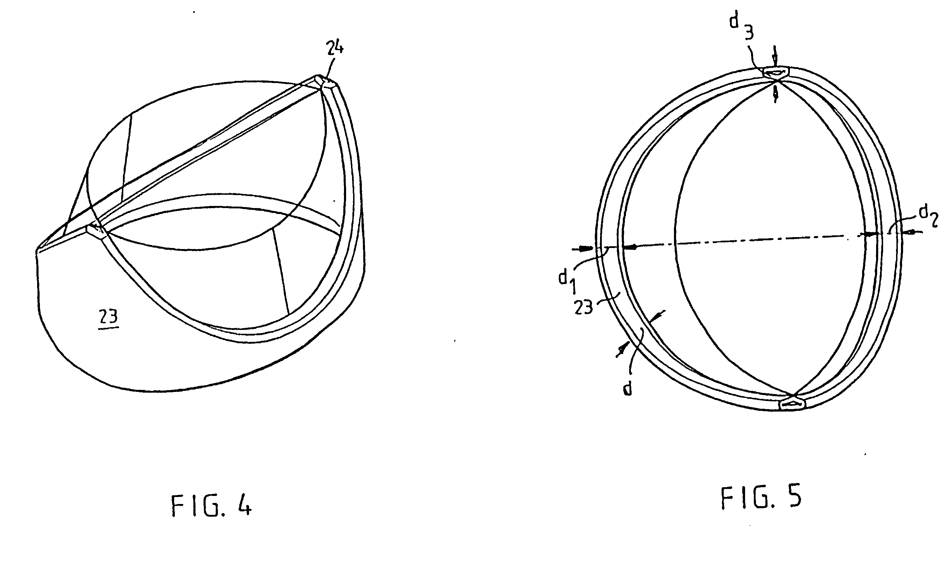

Fig. 4 zeigt eine alternative Ausführungsform einer Mitral-Herzklappe, bei der die vorbeschriebenen Pfosten gar nicht mehr körperlich in Erscheinung treten. Vielmehr sind bei dieser Ausführungsform die Pfosten dickengleich in die Wandungen 23 integriert. Die Wandungen laufen an gegenüberliegenden Enden nach oben hin zu einem stirnseitigen Pfostenende 24 aus, das spitz oder, wie dargestellt, abgeflacht sein kann.

Die Dicke der Wandung d kann vom Basisring zur oberen Kante der Wandungen kontinuierlich abnehmen. Wie aus Fig. 5 näher ersichtlich, sind die Dicken d der Wandungen 23, gemessen in Höhe des Basisringes, im Bereich der Pfosten minimal und wachsen zu einem Höchstwert an. In einem konkreten Ausführungsbeispiel beträgt das Dickenmaß d₁ 2,57 mm, das Dickenmaß d₂ 2,34 mm und das Dickenmaß d₃ (im Bereich beider Pfosten) 1,4 mm.

Bei der Fertigung der beschriebenen Herzklappe können die jeweils gefertigten Segel durch Kleben oder Verschweißen an den Stirnflächen des Stützgehäuses befestigt werden. Alternativ hierzu ist es auch möglich, die Herzklappe mittels der nach dem Stand der Technik bekannten Spritzgußtechnik einschließlich des 2-Komponentenspritzens herzustellen, bei dem zunächst das Stützgehäuse gefertigt und anschließend hieran die Segel durch Spritzgießen angebracht werden. Eine weitere Möglichkeit ist die Anwendung der sogenannten Tauchtechnik. Hierzu wird das z.B. aus Polyamid befestehende Stützgehäuse nach einer Beschichtung mit Polyurethan auf einen entsprechenden Tauchdorn mit Formflächen für die Segel aufgeschoben und anschließend der Tauchdorn mit dem Stützgehäuse in eine flüssige Kunststofflösung (Polyurethan) eingetaucht und so lange taumelnd bewegt, bis die gewünschte Dickenverteilung erreicht ist. Während des Taumelns härtet der Kunststoff aus.

Die Erfindung erstreckt sich auch auf künstliche Blutpumpen (Kunstherzen), Conduitklappenimplantate, Bioprothesen oder mechanische Prothesen und ähnliches, bei denen das Stützgehäuse ein integrierter Bestandteil eines rohrförmigen Gehäuses oder Schlauches ist.

## Patentansprüche

1. Prothetische Mitral-Herzklappe, bestehend aus einem Stützgehäuse (Stent) (10) mit einem Basisring (12), der zwei im wesentlichen in Ringachsrichtung weisende, über bogenförmige, der Befestigung zweier flexibler Segel (11) dienende Wandungen (13,14) verbundene Pfosten (18,19) trägt, deren freie Enden eine Innenauflage für das Segel (11) bilden,
**dadurch gekennzeichnet,**
**daß** der Basisring (12) - in Draufsicht betrachtet - als eine geschlossene unrunde Form aus zwei Halbformen mit unterschiedlicher Krümmung ausgebildet ist, die an zwei Übergangsstellen ineinander übergehen und einer gemeinsamen Längsachse (15), die durch die Übergangsstellen geht und zwei ungleich große halbe Querachsen (16,17), wobei die Pfosten (18,19) auf der Längsachse (15) liegen und die Übergangsstelle von der einen zur anderen Halbform bilden und wobei die Wandung (13) mit geringerer Krümmung ein unter einem zur Basisring-Grundfläche stärker geneigten Winkel angeordnetes flächenkleineres oder murales Segel (11) trägt als die Wandung (14) mit größerer Krümmung.

2. Mitral-Herzklappe nach Anspruch 1, **dadurch gekennzeichnet, daß** die Segelneigung, die durch die Lage der Verbindungslinie des Segels (11) mit der oberen Innenkante der Wandung (13,14) bestimmt ist, zwischen 25° und 45° für das weniger geneigte oder aortale Segel und zwischen 40° und 65° für das stärker geneigte (murale) Segel, jeweils relativ zur Basisgrundfläche, beträgt und gleichzeitig das stärker geneigte Segel um mindestens 5° stärker geneigt ist als das weniger geneigte Segel, so daß die Hauptströmungsrichtung um 10° bis 25°, vorzugsweise um 15° von der Normalen zum muralen Segel geneigt ist.

3. Mitral-Herzklappe nach einem der Ansprüche 1 bis 2, **dadurch gekennzeichnet, daß** die Längen der halben Querachsen (16,17) in einem Verhältnis von 1,5 bis 2,5 : 1 stehen und/oder daß die gemeinsame Längsachse (15) eine Länge zwischen 10 mm und 45 mm aufweist.

4. Mitral-Herzklappe nach einem der Ansprüche 1 bis 3, **dadurch gekennzeichnet, daß** die Pfosten dickengleich in die Wandungen (23) integriert sind, wobei das stirnseitige Pfostenende (24) spitz oder abgeflacht ausläuft.

5. Mitral-Herzklappe nach einem der Ansprüche 1 bis 4, **dadurch gekennzeichnet, daß** die Wandungsdicke (d) vom Basisring zur oberen Kante der Wandungen abnimmt, vorzugsweise kontinuierlich abnimmt.

6. Mitral-Herzklappe nach einem der Ansprüche 1 bis 5, **dadurch gekennzeichnet, daß** die Pfosten (18,19) sich zu ihrem freien Ende zum Stirnflächenmaß verdicken, vorzugsweise kontinuierlich, wobei die Pfosten (18,19) sich zur Basisgrundfläche hin und vor dieser endend keilförmig verjüngen.

7. Mitral-Herzklappe nach einem der Ansprüche 1 bis 6, **dadurch gekennzeichnet, daß** die Dicke der Wandungen (13,14) des Basisringes (12) im Bereich zwischen den Pfosten (18,19), d.h. an der Segelbasis, größer ist als im pfostennahen Bereich, vorzugsweise um einen Faktor 1,4 bis 2,3.

8. Mitral-Herzklappe nach einem der Ansprüche 1 bis 7, **dadurch gekennzeichnet, daß** die Verbindungslinie der Segel (11) mit der oberen Innenkante der Wandungen (13,14) jeweils in einer Ebene liegt.

9. Mitral-Herzklappe nach Anspruch 2, **dadurch gekennzeichnet, daß** die Pfostenlängsachse (21) in Richtung der Hauptströmungsrichtung verläuft.

## Claims

1. Prosthetic mitral heart valve consisting of a support housing (10) with a base ring (12) which carries two posts (18, 19) substantially extending in ring axis direction and connected by curved walls (13, 14), the free ends of the posts forming inner supports for the cusp (11) **characterised in that** the base ring (12) - seen in a top view - is formed as a closed nonround shape consisting of two half shapes with different curvatures merging into each other at two transition areas and with a common longitudinal axis (15) going through the transition areas and two unequal sized half transverse axes (16, 17), whereby the posts (18, 19) lie on the longitudinal axis (15) and form the transition area from one of the half shape to the other half shape and whereby the wall (13) with the smaller curvature carries a smaller area or mural cusp (11) with a higher angle of inclination relative to the base ring basal surface than the wall (14) with greater curvature.

2. Prosthetic mitral heart valve according to claim 1, **characterised in that** the cusp inclination which is determined by the position of the connection line of the cusp (11) with the upper inner edge of the wall portion (13, 14) lies between 25° and 45° for the less inclined or aortal cusp and between 40° and 65° for the more strongly inclined (mural) cusp, each with respect to the base ground surface, and simultaneously the more inclined cusp is inclined at an angle of at least 5° more than the lesser inclined cusp, so that the main flow direction is inclined by about 10° to 25°, preferably about 15° from the normal to the mural cusp.

3. Prosthetic mitral heart valve according to one of claims 1 to 2, **characterised in that** lengths of the half transverse axes (16, 17) is in a ratio of 1.5 to 2.5:1 and/or that the common longitudinal axis (15) has a length between 10 mm and 45 mm.

4. Prosthetic mitral heart valve according to one of claims 1 to 3, **characterised in that** the posts are integrated to be equal in thickness of the wall (23), whereby the end faces of the post ends (24) are pointed or flattened.

5. Prosthetic mitral heart valve according to one of claims 1 to 4, **characterised in that** the wall thickness (d) decreases from the base ring toward the upper edge of the walls preferably continuously.

6. Prosthetic mitral heart valve according to one of claims 1 to 5, **characterised in that** the posts (18, 19) increases in thickness toward their free ends to the end face dimensions, preferably continuously, whereby the posts (18, 19) converge wedge-shaped toward the base ground surface and end before it.

7. Prosthetic mitral heart valve according to one of claims 1 to 6, **characterised in that** the thicknesses of the walls (13, 14) of the base ring (12) in the region between the posts (18, 19) i.e. at the cusp bases, is greater that in the regions close to the posts, preferably by a factor of 1.4 to 2.3.

8. Prosthetic mitral heart valve according to one of the claims 1 to 7, **characterised in that** the connection line of the cusps (11) and the upper inner edge of the walls (13, 14) each lie in a plane.

9. Prosthetic mitral heart valve according to claim 2, **characterised in that** the post longitudinal axes (21) runs in the direction of the main flow direction.

## Revendications

1. Valvule cardiaque mitrale prothétique se composant d'un boîtier de support (stent) (10) avec un anneau de base (12) qui porte deux poteaux (18, 19) qui montrent pour l'essentiel dans la direction de l'axe de l'anneau et sont reliés par l'intermédiaire de parois en arc (13, 14) qui servent à la fixation de deux voiles flexibles (11), les extrémités libres des poteaux formant un appui intérieur pour le voile (11),
**caractérisée par le fait**
**que** l'anneau de base (12) - vu dans une vue de dessus - est réalisé comme une forme fermée non ronde se composant de deux demi-formes à courbure différente qui débouchent l'une dans l'autre à deux endroits de transition et avec un axe longitudinal commun (15) qui passe à travers les endroits de transition et avec deux demi-axes transversaux (16, 17) à grandeur inégale, les poteaux (18, 19) étant situés sur l'axe longitudinal (15) et formant l'endroit de transition de l'une des demi-formes à l'autre, et la paroi (13) avec la courbure plus faible portant un voile (11) à surface plus petite ou mural disposé à un angle plus fortement incliné par rapport à la surface de base de l'anneau de base, que la paroi (14) avec la courbure plus grande.

2. Valvule cardiaque mitrale selon la revendication 1, **caractérisée par le fait que** l'inclinaison du voile, qui est déterminée par la position de la ligne de jonction du voile (11) avec l'arête intérieure supérieure de la paroi (13, 14), est comprise entre 25° et 45° pour le voile à inclinaison plus faible ou aortique et entre 40° et 65° pour le voile (mural) à inclinaison plus forte, respectivement par rapport à la surface de base, et que, en même temps, le voile à inclinaison plus forte est incliné d'au moins 5° plus fortement que le voile à inclinaison plus faible, de sorte que le sens principal d'écoulement est incliné de 10° à 25°, de préférence de 15° de la normale au voile mural.

3. Valvule cardiaque mitrale selon l'une des revendications 1 à 2, **caractérisée par le fait que** les longueurs des demi-axes transversaux (16, 17) sont dans un rapport allant de 1,5 : 1 à 2,5 : 1 et/ou que l'axe longitudinal commun (15) présente une longueur comprise entre 10 mm et 45 mm.

4. Valvule cardiaque mitrale selon l'une des revendications 1 à 3, **caractérisée par le fait que** les poteaux sont intégrés à épaisseur égale dans les parois (23), l'extrémité de front (24) du poteau se termine en pointe ou de façon aplatie.

5. Valvule cardiaque mitrale selon l'une des revendications 1 à 4, **caractérisée par le fait que** l'épaisseur de paroi (d) diminue de l'anneau de base à l'arête supérieure des parois, de préférence de façon continue.

6. Valvule cardiaque mitrale selon l'une des revendications 1 à 5, **caractérisée par le fait que** les poteaux (18, 19) s'épaississent, de préférence de façon continue, vers leur extrémité libre de manière à avoir la dimension de la surface frontale, les poteaux (18, 19) se rétrécissant en forme de coin vers la surface de base et se terminant devant elle.

7. Valvule cardiaque mitrale selon l'une des revendications 1 à 6, **caractérisée par le fait que** l'épaisseur des parois (13, 14) de l'anneau de base (12) dans la zone entre les poteaux (18, 19), c'est-à-dire sur la base de voile, est plus grande que dans la zone près des poteaux, de préférence d'un facteur de 1,4 à 2,3.

8. Valvule cardiaque mitrale selon l'une des revendications 1 à 7, **caractérisée par le fait que** la ligne de jonction des voiles (11) avec l'arête intérieure supérieure des parois (13, 14) est située respectivement dans un plan.

9. Valvule cardiaque mitrale selon la revendication 2, **caractérisée par le fait que** l'axe longitudinal (21) du poteau s'étend dans la direction du sens principal d'écoulement.
